# FASCICULE DE BREVET EUROPEEN

(11) **EP 3 145 579 B1**
(45) Date de publication et mention de la délivrance du brevet: **04.10.2023**
(21) Numéro de dépôt: 15732353.6
(22) Date de dépôt: 11.05.2015
(51) Int. Cl.: A61N 1/04, A61N 1/375, A61F 13/02, A61F 13/00

(54) **DISPOSITIF CUTANÉ COMPRENANT UNE ZONE FLEXIBLE ET DES MOYENS DE LIMITATION DE LA FLEXION**
GERÄT FÜR DIE HAUT MIT EINEM BIEGBAREN GEBIET UND MIT MITTELN ZUR BEGRENZUNG DER BIEGBARKEIT
DEVICE FOR THE SKIN COMPRISING A FLEXIBLE AREA AND MEANS FOR LIMITING THE FLEXIBILITY

(30) Priorité: 19.05.2014 FR 1454461
(43) Date de publication de la demande: 29.03.2017
(73) Titulaire: Commissariat à l'Énergie Atomique et aux Énergies Alternatives, 75015 Paris (FR)
(72) Inventeur: KARST, Nicolas, F-57600 Folkling (FR); EMIEUX, Fabrice, F-38340 Voreppe (FR); PERRAUD, Simon, F-83150 Bandol (FR)
(74) Mandataire: Gevers & Orès
(86) Numéro de dépôt international: PCT/IB2015/053456
(87) Numéro de publication internationale: WO 2015/177676

(56) Documents cités:
- US-A- 3 472 233
- US-A1- 2010 280 573
- US-A1- 2013 313 713
- US-B1- 6 725 090

## Description

L'invention, telle que définie dans la revendication 1, concerne le domaine technique des dispositifs destinés à être fixés sur la peau d'un utilisateur.

Il s'agit notamment de dispositifs médicaux, comme des électrodes cutanées pour la mesure de paramètres physiologiques ou pour l'électrostimulation, ou encore des générateurs d'impulsions pour l'électrostimulation.

En effet, afin de pouvoir contrôler un certain nombre de paramètres physiologiques en temps réel, les médecins font de plus en plus appel à des dispositifs médicaux portables afin que le patient ne soit pas contraint de rester dans une structure médicalisée durant la période de surveillance. En plus d'un confort accru pour le patient, cela permet de diminuer les coûts liés à l'utilisation de ces structures médicalisées et également, à terme, d'accompagner efficacement au quotidien les patients face à leurs maladies.

Afin d'améliorer encore le confort et l'ergonomie de ces dispositifs médicaux, il a été proposé des dispositifs sous la forme de patchs généralement autonomes en énergie et destinés à être portés directement sur la peau du patient. On peut notamment citer l'utilisation de patchs permettant de réaliser des électrocardiogrammes ou de générer des impulsions électriques pour l'électrostimulation des nerfs utilisées dans le traitement de la douleur.

Afin d'être le plus ergonomique possible et de se conformer au mieux aux différentes parties du corps humain sur lesquelles il est amené à être positionné, le dispositif doit être le plus fin et flexible possible.

Cependant, il est aussi nécessaire de protéger les éléments du dispositif pouvant être détériorés par des flexions répétées, comme les composants électroniques, les batteries, les circuits imprimés ou les pistes métalliques. C'est pourquoi ces éléments sont généralement positionnés dans des structures rigides et seules les électrodes sont flexibles.

Ainsi, le dispositif décrit dans le document US2013/0096641 est composé d'un boitier rigide intégrant l'électronique et la source d'énergie permettant de délivrer des impulsions électriques et qui est monté sur un patch adhésif permettant de maintenir le boitier en contact avec la peau.

De même, dans le dispositif décrit dans le document WO 2013/106644, les éléments sensibles à la flexion sont protégés dans un boitier rigide, seules les parties servant d'électrodes étant flexibles.

Dans ces dispositifs, la présence d'un boîtier rigide permet de protéger efficacement les éléments sensibles à la flexion mais limite grandement le confort et l'ergonomie des dispositifs.

L'invention a pour objet de pallier ces inconvénients en proposant un dispositif destiné à être fixé sur la peau d'un utilisateur qui soit à la fois fin, flexible et robuste, en assurant la protection des composants du dispositif susceptibles d'être endommagés notamment par les contraintes générées par la mise en flexion du dispositif.

Ainsi, l'invention a pour objet un dispositif destiné à être fixé sur la peau d'un utilisateur, le dispositif étant sensiblement plan et comprenant au moins une zone flexible dans laquelle le dispositif peut être fléchi par rapport à un axe situé dans le plan du dispositif et dans laquelle sont situés des composants sensibles à la flexion, le dispositif comprenant des moyens de limitation de la flexion pour limiter la flexion de ladite au moins une zone flexible du dispositif par rapport à au moins un axe situé dans le plan du dispositif.

Dans un premier mode de réalisation, lesdits moyens comprennent une partie centrale allongée, de part et d'autre de laquelle s'étendent des parties en saillie définissant entre elles des renfoncements.

Les moyens de limitation de la flexion définissent un rayon de courbure déterminé au niveau d'une zone de flexion. La valeur minimale du rayon de courbure dépend de la largeur L correspondant à l'espacement entre deux parties en saillie et de la hauteur H des parties en saillie.

Afin d'obtenir cet effet de limitation du rayon de courbure, il convient que L et H soient choisis de telle sorte que L < π H et, de préférence, L < 2 H.

Dans une première variante de réalisation, lesdits moyens sont situés dans une partie sensiblement centrale du dispositif.

Dans une deuxième variante de réalisation, lesdits moyens sont situés le long d'au moins une partie de la périphérie du dispositif.

Dans une troisième variante de réalisation, lesdits moyens comportent une partie située en périphérie du dispositif et une partie s'étendant sensiblement au centre du dispositif.

De façon avantageuse, la surface occupée par lesdits moyens est inférieure à 50% de la surface du dispositif et, de préférence, inférieure à 10% de cette surface.

Dans un deuxième mode de réalisation du dispositif, les moyens pour limiter la flexion sont formés par le boîtier d'encapsulation du dispositif.

De façon préférée, le dispositif comprend avantageusement au moins deux parties rigides reliées par une zone flexible, dans laquelle sont situés des composants sensibles à la flexion, chacune desdites parties rigides comprenant au moins deux parois du boîtier d'encapsulation, les parois en regard de deux parties rigides adjacentes constituant des moyens de limitation de la flexion selon un axe s'étendant dans ladite zone flexible et parallèle auxdites parois.

De façon avantageuse, la hauteur (H) des parois et la largeur (L) de l'espacement entre deux parois en regard sont telles que L < πH et, de préférence, L < 2H.

Dans une première variante de réalisation, le dispositif comprend une pluralité de parties rigides reliées entre elles deux à deux par une zone flexible et s'étendant selon une direction déterminée.

Dans une deuxième variante de réalisation, au moins quatre zones rigides sont reliées entre elles par au moins deux zones flexibles s'étendant selon deux directions différentes.

L'invention sera mieux comprise et d'autres buts, avantages et caractéristiques de celle-ci apparaîtront plus clairement à la lecture de la description qui suit et qui est faite au regard des dessins annexés, sur lesquels :
- la figure 1 est une vue de dessus d'un premier exemple de réalisation d'un dispositif selon l'invention,
- la figure 2 comprend les figures 2A et 28 qui sont des vues en perspective d'un élément plan flexible en l'absence et en présence d'une flexion par rapport à axe parallèle x,
- la figure 3 est une vue de dessus d'un troisième mode de réalisation d'un dispositif selon l'invention,
- la figure 4 est une vue de dessus d'un quatrième mode de réalisation d'un dispositif selon l'invention qui est une variante du dispositif illustré à la figure 3,
- la figure 5 est une vue de dessus d'un cinquième mode de réalisation du dispositif selon l'invention,
- la figure 6 est une vue de dessus d'un sixième mode de réalisation du dispositif selon l'invention qui est une variante du dispositif illustré à la figure 5,
- la figure 7 est une vue de dessus d'un septième mode de réalisation du dispositif selon l'invention,
- la figure 8 comprend les figures 8A, 8B et 8C, la figure 8A étant une vue de dessus d'une partie d'un huitième mode de réalisation du dispositif selon l'invention, la figure 8B étant une vue en coupe selon un axe parallèle à l'axe VIII-VIII de la figure 8A et la figure 8C étant une vue en coupe selon le même axe de l'ensemble du dispositif,
- la figure 9 comprend les figures 9A et 9B correspondant à une vue de dessus et une vue en coupe selon la ligne A-A d'un neuvième mode de réalisation du dispositif selon l'invention,
- la figure 10 comprend les figures 10A et 10B qui représentent, vu de dessus et vu en coupe selon l'axe X-X, un dixième mode de réalisation du dispositif selon l'invention, et
- la figure 11 comprend les figures 11A et 11B qui représentent vue de dessus et vue de côté, une variante de réalisation d'un élément de limitation de la flexion tel qu'illustré à la figure 3.

Les éléments communs aux différentes figures seront illustrés par les mêmes références.

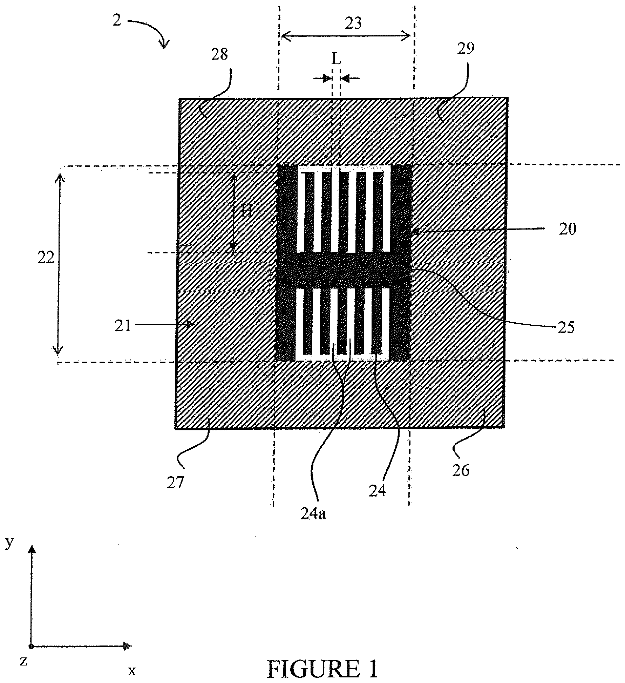

La figure 1 illustre, vu de dessus, un premier exemple d'un dispositif selon l'invention.

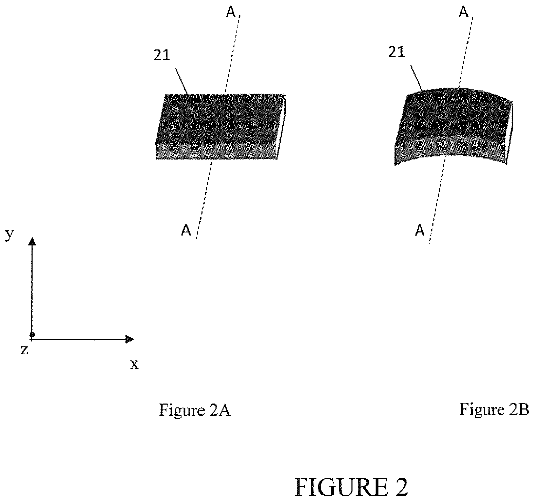

Ce dispositif 2 comprend un élément 21 plan, ici de forme carrée, et contenu dans un plan xy représenté également sur la figure 2. Cet élément 21 comporte des éléments sensibles à la flexion.

Les éléments sensibles à la flexion peuvent notamment être des composants électroniques, des pistes métalliques, des circuits imprimés ou encore des sources d'énergie.

L'élément 21 est évidé et reçoit, de façon sensiblement centrale, un élément de limitation de la flexion 20. Ledit élément 20 est susceptible de limiter la flexion du dispositif.

L'élément 21 permet de définir les zones 22 et 23 de l'élément 21, toutes deux sont situées dans le prolongement de l'élément 20, selon l'axe x et selon l'axe y, dans deux directions opposées. Les autres zones de l'élément 21 sont identifiées par les références 26 à 28. Elles sont situées dans les angles de l'élément 21 et sont délimitées par les lignes en pointillés indiquées sur la figure 1.

Cet élément 20 de limitation de la flexion comporte une partie centrale 25 allongée, de part et d'autre de laquelle s'étendent des parties en saillie 24 définissant des renfoncements 24a. Dans l'exemple illustré à la figure 1 et dans une vue de dessus, les parties en saillie s'étendent dans le plan xy.

Bien entendu, l'élément 20 présente une certaine épaisseur, tout comme l'élément 21. Ils ont donc également une composante selon un axe z perpendiculaire au plan xy, correspondant à cette épaisseur. Ainsi, la partie centrale s'étend selon un plan xz.

Grâce à sa structure, l'élément 20 de limitation de la flexion empêchera complètement la flexion du dispositif 2 par rapport à un axe quelconque parallèle à l'axe x, au niveau de la zone 22.

La figure 2 illustre l'effet d'une flexion autour d'un axe AA parallèle à l'axe y sur l'élément 21, lequel s'étend dans le plan xy.

Cet élément 20 de limitation de la flexion permettra également de limiter la flexion à un rayon de courbure déterminé, au niveau de la zone 23, par rapport à un axe quelconque parallèle à l'axe y, et donc de protéger des composants sensibles à la flexion, situés au niveau de la zone de flexion 23, d'une flexion trop importante par rapport à un axe parallèle à l'axe y qui pourrait les endommager.

La limitation du rayon de courbure est obtenue en choisissant de façon appropriée la largeur L correspondant à l'espacement entre deux parties en saillie et la hauteur H de ces parties en saillie.

Ainsi, L et H sont choisies de telle sorte que L < πH et, de préférence, L < 2H.

Cependant, dans cette configuration, les éléments sensibles à la flexion ne peuvent pas être efficacement protégés d'une flexion par rapport à un axe quelconque parallèle à l'axe y au niveau des zones 28 et 29, ni au niveau de la zone 23. De même, les éléments sensibles à la flexion ne peuvent pas être efficacement protégés d'une flexion selon un axe quelconque parallèle à l'axe x au niveau des zones 26 et 27.

De plus, pour protéger des zones présentant une surface importante, l'élément de limitation de la flexion 20 illustré à la figure 2 doit occuper une surface également importante, ce qui diminue d'autant la surface occupée par les différents composants du dispositif.

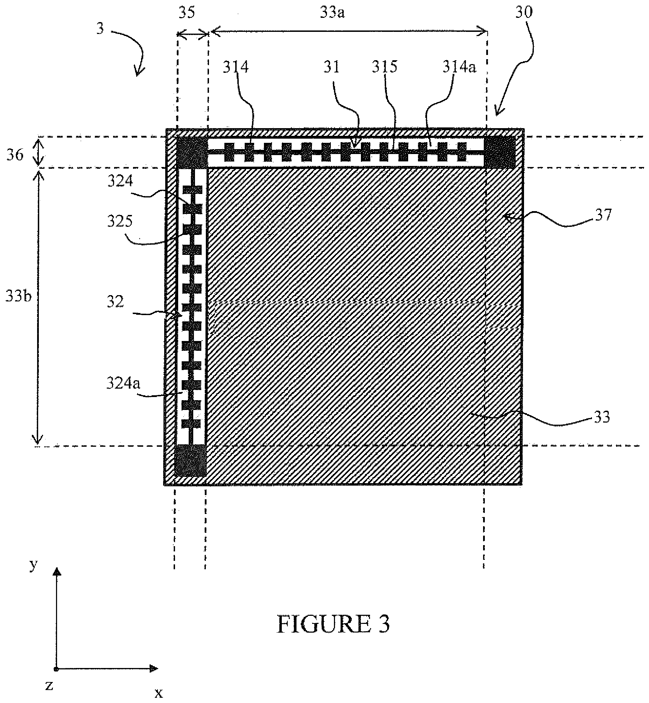

Il est maintenant fait référence à la figure 3 qui illustre, vu de dessus, un autre mode de réalisation du dispositif selon l'invention permettant d'assurer une meilleure protection des composants du dispositif, à l'encontre d'une flexion, quel que soit l'axe par rapport auquel elle se produit.

Dans ce mode de réalisation, le dispositif 3 est encore sensiblement plan et s'étend selon le plan xy. Il présente également une certaine épaisseur et donc une composante selon l'axe z perpendiculaire au plan xy. Il présente une forme de préférence carrée et comporte des éléments sensibles à la flexion sur sa surface 37.

Il comporte un élément 30 destiné à limiter la flexion.

Dans cet exemple de réalisation, cet élément 30 de limitation de la flexion est composé de deux parties 31 et 32 s'étendant dans des directions différentes.

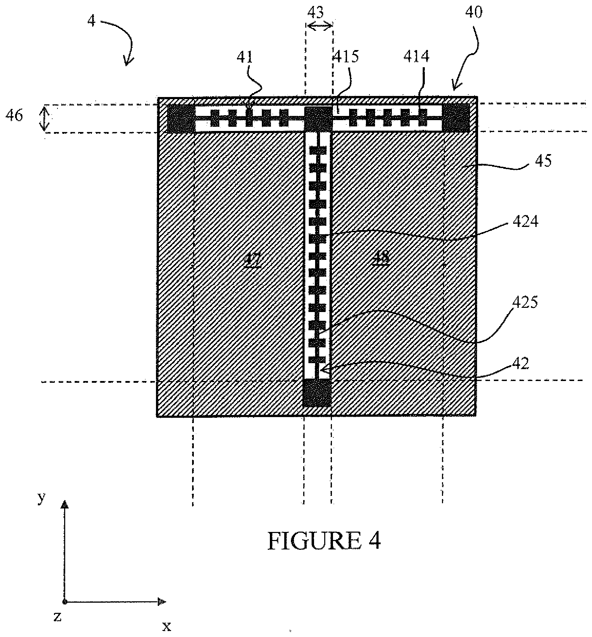

Dans l'exemple illustré à la figure 3, ces deux parties 31 et 32 sont perpendiculaires et s'étendent selon deux côtés adjacents du dispositif 3. D'autres configurations pourraient être envisagées, comme celles décrites en référence aux figures 4 à 7.

Chaque partie 31 et 32 de l'élément 30 de limitation de la flexion présente la même structure générale que l'élément de limitation de la flexion 20 illustré à la figure 1.

Ainsi, chacun d'eux comporte une partie centrale 315, 325 allongée, de part et d'autre de laquelle s'étendent des parties en saillie 314, 324, définissant des renfoncements 314a, 324a.

Grâce à cette structure particulière, l'élément 30 permet de limiter la flexion du dispositif 3 sur toute sa surface 37, par rapport à un axe quelconque, parallèle à l'axe x ou à l'axe y.

L'élément 30 définit une zone 33 sensiblement carrée et dont les deux côtés sont identifiés par les références 33a et 33b, ainsi que deux zones 35 et 36 correspondant à la partie de la surface 37 occupée par les parties 32 et 31 de l'élément 30. Les deux côtés 33a et 33b correspondent à la longueur des parties 31 et 32 qui ont un rôle actif dans la limitation de la flexion, c'est-à-dire à la longueur de la partie centrale.

Les zones 35 et 36 ne sont pas destinées à recevoir des composants, dans la mesure ou elles sont occupées par l'élément 30.

La partie 31 de l'élément 30 permet de limiter la flexion du dispositif 3 à un rayon de courbure déterminé au niveau de la zone 33, par rapport à un axe quelconque parallèle à l'axe y. La partie 32 permet également de limiter la flexion du dispositif 3 à un rayon de courbure déterminé au niveau de la zone 33, par rapport à un axe quelconque parallèle à l'axe x. Il suffit pour cela que la hauteur H et l'espacement L des parties en saillie soient telles que L < πH et, de préférence, L < 2H.

Enfin, l'élément 30 empêche totalement la flexion au niveau de la zone 35 par rapport à un axe quelconque parallèle à l'axe y, ainsi que la flexion au niveau de la zone 36, par rapport à un axe quelconque parallèle à l'axe x.

Avantageusement, la surface occupée par l'élément 30 de limitation de la flexion sera inférieure à 50% de la surface totale du dispositif 3 et de préférence inférieure à 10%.

Avec cet élément 30 de limitation de la flexion, la surface des zones où la flexion est impossible est relativement limitée et sera définie par la surface occupée par l'élément 30.

Ceci présente un avantage par rapport au mode de réalisation de la figure 1. En effet, le dispositif est destiné à être placé sur le corps d'un utilisateur. Il est donc avantageux de limiter au maximum la surface occupée par les zones rendant totalement impossible la flexion.

Ainsi pour un dispositif de 5 × 5 cm², la surface occupée par l'élément 30 de limitation de la flexion pourra être limitée à 15% pour des parties 31 et 32 dont la largeur est d'environ 0.5 cm.

Le rayon de courbure minimal par rapport à un axe quelconque parallèle à l'axe x et par rapport à un axe quelconque parallèle à l'axe y sera inférieur à 5 cm, de préférence inférieur à 2 cm.

Il est maintenant fait référence à la figure 4 qui illustre une variante de la figure 3. Le dispositif 4 est, là encore, de forme carrée. Il s'étend selon le plan xy et présente une épaisseur selon l'axe z perpendiculaire au plan xy.

Dans ce mode de réalisation, le dispositif 4 comporte un élément 40 de limitation de la flexion, constitué de deux éléments 41 et 42. Il est disposé à l'intérieur du dispositif 4.

Ces deux éléments 41 et 42 s'étendent dans deux directions différentes, ici deux directions perpendiculaires.

Un des éléments 41 s'étend selon un des côtés du dispositif 4, tandis que l'autre élément 42 s'étend au travers du dispositif 4, sensiblement en position centrale.

Ainsi, cet élément central 42 permet de définir deux zones 47 et 48 de la surface totale du dispositif. Chacune de ces zones est dans le prolongement de l'élément latéral 41 selon l'axe y.

Le dispositif comporte, sur l'ensemble de sa surface, des éléments sensibles à la flexion, à l'exception des zones 43 et 46 occupées par l'élément 40.

Chacun de ces éléments 41, 42 comporte une partie centrale 415, 425, de part et d'autre de laquelle s'étendent des parties en saillie 414, 424. La partie centrale 415 s'étend selon le plan xz et la partie centrale 425 s'étend selon le plan yz.

Dans la configuration illustrée à la figure 4, l'élément 40 de limitation de la flexion permet de protéger les éléments sensibles à la flexion en limitant la flexion du dispositif 4 au niveau de l'ensemble de la surface du dispositif 4 par rapport à un axe quelconque parallèle à l'axe x ou à l'axe y.

L'élément 40 permet, grâce à l'élément 41, de limiter la flexion du dispositif 4 à un rayon de courbure déterminé au niveau des zones 47 et 48 par rapport à un axe parallèle à l'axe y. L'élément 40 permet également de limiter la flexion du dispositif 4 à un rayon de courbure déterminé au niveau des zones 47 et 48 par rapport à un axe parallèle à l'axe x, grâce à l'élément 42. Il suffit pour cela que la hauteur H et l'espacement L des parties en saillie soient telles que L < πH et, de préférence, L < 2H.

L'élément 40 empêche complètement la flexion au niveau de la zone 43 selon un axe parallèle à l'axe y, ainsi que la flexion au niveau de la zone 46 par rapport à un axe quelconque parallèle à l'axe x. Cependant, l'élément 40 est susceptible de se conformer à une partie du corps humain.

En effet, la surface occupée par l'élément 40 sera toujours inférieure à 50% de la surface totale du dispositif 4, de préférence inférieure à 10%.

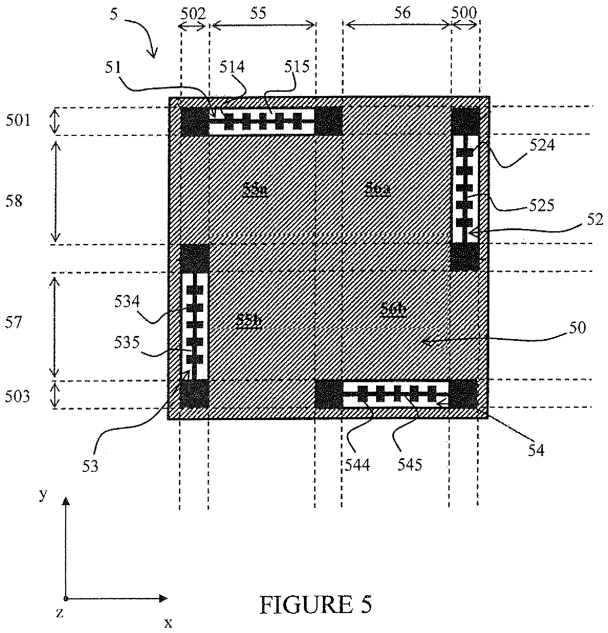

Il est maintenant fait référence à la figure 5 qui illustre un autre mode de réalisation du dispositif selon l'invention.

Le dispositif 5 présente là encore une forme de préférence carrée et sensiblement plane. Il définit une surface 50 comportant des composants sensibles à la flexion qui s'étend dans le plan xy. Le dispositif présente également une épaisseur selon l'axe z perpendiculaire au plan xy.

Dans le dispositif 5, sont prévus plusieurs éléments limitant la flexion, ici au nombre de quatre.

Les éléments 51, 52, 53 et 54 présentent ici la même structure générale, à savoir une partie centrale 515, 525, 535 et 545 allongée, de part et d'autre de laquelle s'étendent des parties en saillie 514, 524, 534 et 544.

Chacun de ces éléments 51 à 54 est disposé parallèlement à un côté du dispositif 5, de telle sorte que sa partie centrale soit sensiblement parallèle à ce côté.

De plus, dans l'exemple illustré à la figure 5, chacun des éléments 51 à 54 présente une longueur inférieure à celle d'un côté du dispositif, de telle sorte que des espaces sont ménagés entre chacun d'eux.

Ces éléments de limitation de la flexion 51, 52, 53 et 54 définissent plusieurs zones dans la surface 50 : les zones 55 et 56 dans le prolongement des éléments 51 et 54 selon l'axe y et les zones 57 et 58 dans le prolongement des éléments 52 et 53 selon l'axe x.

En pratique, ces zones ont des parties communes. Ainsi, la zone 55 comprend les zones 55a et 55b, la zone 55a étant commune avec la zone 58 et la zone 55b étant commune avec la zone 57. De même, la zone 56 comprend les zones 56a et 56b, la zone 56a étant commune avec la zone 58 et la zone 56b étant commune avec la zone 57.

Par ailleurs, les zones 500, 501, 502 et 503 correspondent à un côté de la surface occupé partiellement par un des éléments 51 à 54.

La présence des éléments 51 à 54 permet de protéger les éléments sensibles à la flexion en limitant la flexion du dispositif 5 au niveau de l'ensemble de la surface 50 du dispositif, selon un axe quelconque parallèle à l'axe x ou à l'axe y.

L'élément 51 de limitation de la flexion permet de limiter la flexion du dispositif 5 à un rayon de courbure déterminé au niveau de la zone 55 par rapport à un axe parallèle à l'axe y.

L'élément 51 empêchera complètement la flexion au niveau de la zone 501 par rapport à un axe quelconque parallèle à l'axe x.

L'élément 54 de limitation de la flexion permet de limiter la flexion du dispositif 5 à un rayon de courbure déterminé au niveau de la zone 56 selon un axe quelconque parallèle à l'axe y.

L'élément 54 empêche complètement la flexion au niveau de la zone 503 par rapport à un axe quelconque parallèle à l'axe x.

L'élément 53 de limitation de la flexion permet de limiter la flexion du dispositif 5 à un rayon de courbure déterminé au niveau de la zone 57 par rapport à un axe quelconque parallèle à l'axe x.

L'élément 53 empêche complètement la flexion au niveau de la zone 502 selon un axe quelconque parallèle à l'axe y.

L'élément 52 de limitation de la flexion permet de limiter la flexion du dispositif 5 à un rayon de courbure déterminé au niveau de la zone 58 selon un axe quelconque parallèle à l'axe x. L'élément 52 empêche complètement la flexion au niveau de la zone 500 selon un axe quelconque parallèle à l'axe y.

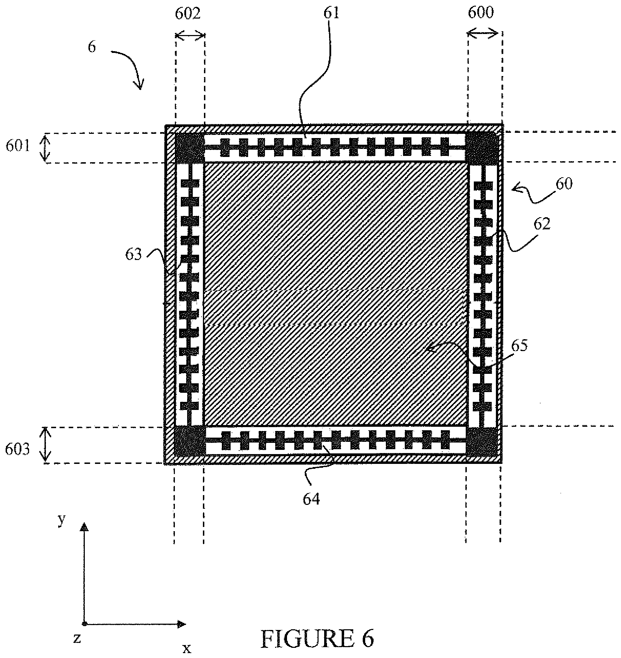

Il est maintenant fait référence à la figure 6 qui illustre une variante de réalisation du dispositif illustré à la figure 5.

Le dispositif 6 présente encore ici une forme sensiblement carrée et plan. Il définit, dans le plan xy, une surface 65.

Selon cette variante, le dispositif 6 comporte un élément 60 de limitation de la flexion qui est constitué de quatre parties 61, 62, 63 et 64. Chacune de ces parties est disposée le long d'un des côtés du dispositif et présente une longueur légèrement inférieure à celle de chaque côté. Ainsi, l'élément 60 constitue en pratique un seul élément constitué de parties orthogonales deux à deux et disposées à la périphérie du dispositif 6.

La structure générale des éléments 61 à 64 ne sera pas décrite de nouveau en détail puisqu'elle est similaire à celles des dispositifs 51 à 54.

L'élément 60 permet de limiter la flexion à un rayon de courbure déterminé sur l'ensemble de la surface 65 du dispositif non occupée par les éléments 61 à 64 et ce, que la flexion intervienne selon un axe parallèle à l'axe x ou à l'axe y.

De plus, chacun des éléments 61 et 64 empêche complètement la flexion au niveau des zones 601 et 603, correspondant aux parties de la surface occupées par ces éléments 61 et 64, par rapport à un axe quelconque parallèle à l'axe x et chacun des éléments 62, 63 empêche complètement la flexion au niveau des zones 600 et 602 occupées par ces éléments par rapport à un axe quelconque parallèle à l'axe y.

L'invention n'est pas limitée à la structure générale d'un élément de limitation de la flexion qui a été décrite en référence aux figures 1 à 6. D'autres éléments permettant de limiter la flexion pourront être utilisés. En effet, on pourra imaginer modifier la répartition, la forme et les dimensions des parties en saillie et des renfoncements de ces éléments.

En particulier, un autre élément de limitation de la flexion peut comporter une partie centrale et des parties en saillie, la partie centrale présentant des orientations différentes.

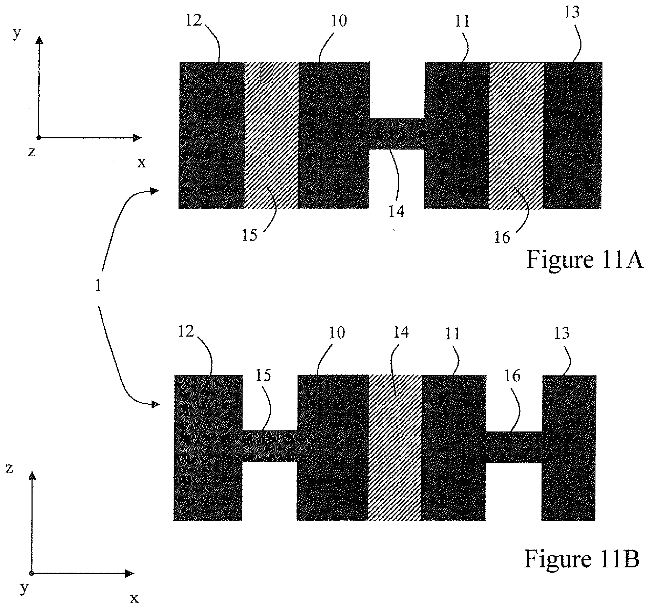

Cette variante de réalisation est illustrée sur les figures 11A et 11B.

La figure 11A est une vue de dessus (ou dans le plan xy) d'un élément 1 de limitation de la flexion. Il comporte quatre parties en saillie 10 à 13 reliées deux à deux par une partie centrale 14 à 16, ces parties centrales n'ayant pas toutes la même orientation.

Ainsi, les parties centrales 15 et 16 s'étendent dans le plan xy, tandis que la partie centrale 14 s'étend selon le plan xz perpendiculaire au plan xy.

La figure 11B illustre ce même élément 1, vu de côté (ou dans le plan xz).

De plus, les éléments de limitation de la flexion peuvent être conçus pour limiter la flexion à des rayons de courbure différents les uns des autres.

Enfin, dans tous les exemples de réalisation, le dispositif peut consister en un substrat ou en un boîtier, l'un et l'autre comportant des éléments sensibles à la flexion.

De plus, l'invention n'est pas limitée aux emplacements particuliers des éléments limitant la flexion qui ont été décrits en référence à ces figures. Ces éléments pourraient être disposés différemment et toujours remplir leur fonction, dans la mesure où l'axe de flexion ne passe pas par la partie centrale des éléments.

Dans la description qui précède, le dispositif selon l'invention présente une forme plane et carrée. Cependant, l'invention n'est pas limitée à ce mode de réalisation et le dispositif pourrait prendre d'autres formes.

Ainsi, le dispositif peut se présenter par exemple sous la forme d'un rectangle, d'un triangle ou d'un disque. Le choix de la forme du dispositif pourra dépendre de différents critères comme la morphologie de la personne ou la zone du corps humain à stimuler électriquement.

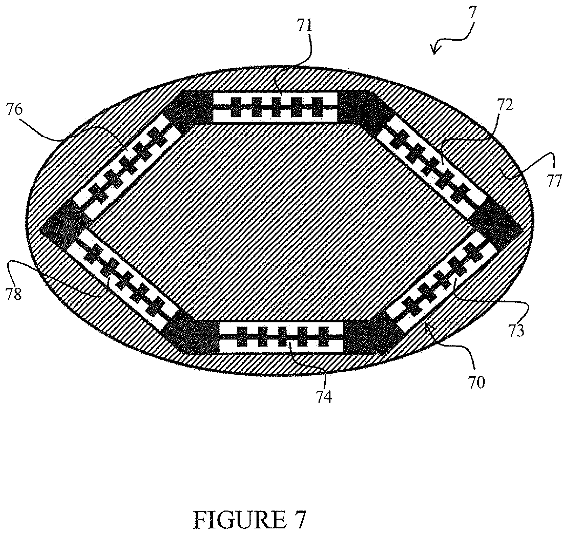

Un exemple de dispositif de forme ovale est représenté à la figure 7.

Sur la surface 77 du dispositif 7, sont prévus des composants sensibles à la flexion.

Par ailleurs, un élément 70 de limitation de la flexion permettant de limiter la flexion est prévu dans le dispositif 7.

Cet élément 70 de limitation de la flexion est ici composé de six parties 71 à 76. Ces six parties sont, en pratique, reliées les unes aux autres de façon à ne former qu'un seul élément.

Ces six parties forment ainsi un hexagone. Chacune d'elles présente une configuration similaire à celle par exemple de la partie 51 illustrée à la figure 5 et qui ne sera pas décrite de nouveau en détail.

Le nombre et la longueur des différentes parties pourraient être modifiés pour que l'élément de limitation de la flexion soit disposé plus proche de la périphérie de la surface 77, de façon à augmenter l'espace disponible pouvant recevoir des éléments sensibles à la flexion.

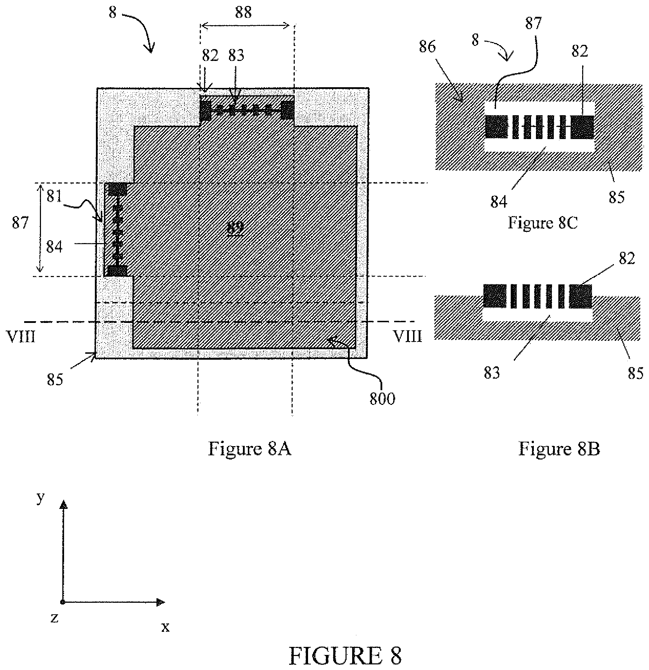

Il est maintenant fait référence à la figure 8 qui illustre une variante dans laquelle les éléments de limitation de la flexion sont intégrés à l'intérieur du packaging (ou boitier d'encapsulation) dans lequel sont logés les éléments nécessaires à la réalisation d'un dispositif cutané selon l'invention (batteries, composants électroniques, pistes métalliques...).

Comme cela apparaîtra au regard de la figure 8, cette configuration présente l'avantage que ces éléments de limitation de la flexion ne sont pas visibles de l'utilisateur.

Ainsi, dans ce mode de réalisation, le boîtier d'encapsulation 8 comporte deux parties 85 et 86.

Il est tout d'abord fait référence à la figure 8A qui est une vue de dessus de la première partie 85.

Ainsi, deux cavités 83 et 84 sont présentes au sein du boîtier 8, dans lesquelles peuvent être insérés des éléments 81 et 82 constitutifs du dispositif de limitation de la flexion.

La figure 8B est une vue en coupe selon un axe parallèle à l'axe VIII-VIII et passant par la cavité 83.

Cette figure 8B illustre un élément 82 de limitation de la flexion au sein de la cavité 83 aménagée dans la première partie 85 du boîtier.

La figure 8C est une vue en coupe selon le même axe que la figure 88, du packaging, composé des deux parties 85 et 86.

La figure 8C montre que la deuxième partie 86 comporte également une cavité 87 susceptible d'accueillir l'élément 82 de limitation de la flexion.

Par ailleurs, à l'intérieur du boîtier 8, est également définie une cavité dans laquelle est disposé un dispositif sensiblement plan 800 qui comporte des éléments sensibles à la flexion.

Afin de diminuer l'épaisseur du boîtier 8, le ou les éléments permettant de limiter la flexion peuvent être en contact avec les parois des deux parties 85 et 86 du boîtier, les cavités étant alors dimensionnées pour recevoir un élément, sans qu'un espace libre ne subsiste.

L'élément 81 permet de définir une zone 87 du dispositif 800, dans son prolongement selon l'axe x, et l'élément 82 permet de définir une zone 88, dans son prolongement selon l'axe y.

Ainsi, l'élément 81 permettra de limiter la flexion au niveau de la zone 87 par rapport à un axe quelconque parallèle à l'axe x et l'élément 82 permettra de limiter la flexion au niveau de la zone 88 par rapport à un axe quelconque parallèle à l'axe y. En pratique, dans la zone 89 correspondant à l'intersection des deux zones 87 et 88, les composants sont protégés des effets d'une flexion par rapport à un axe parallèle à l'axe x ou à l'axe y.

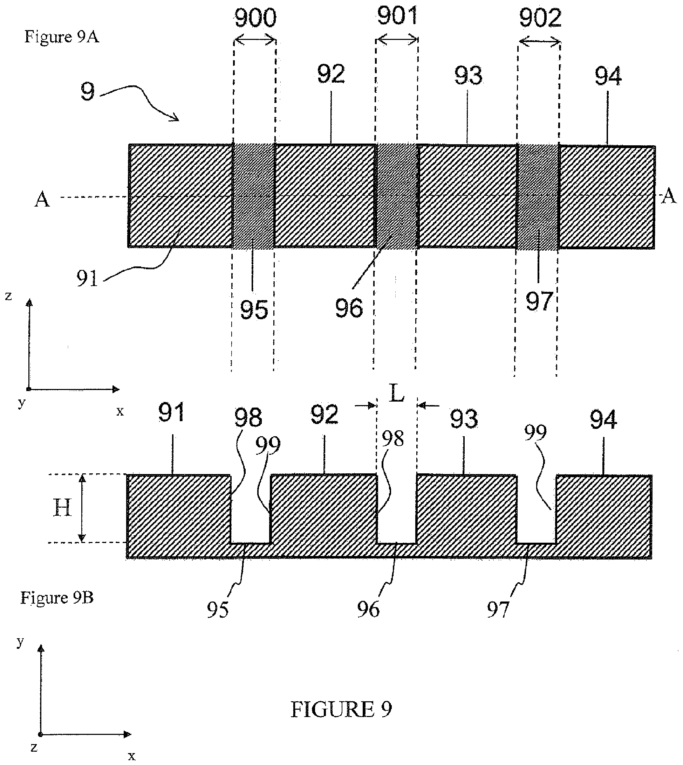

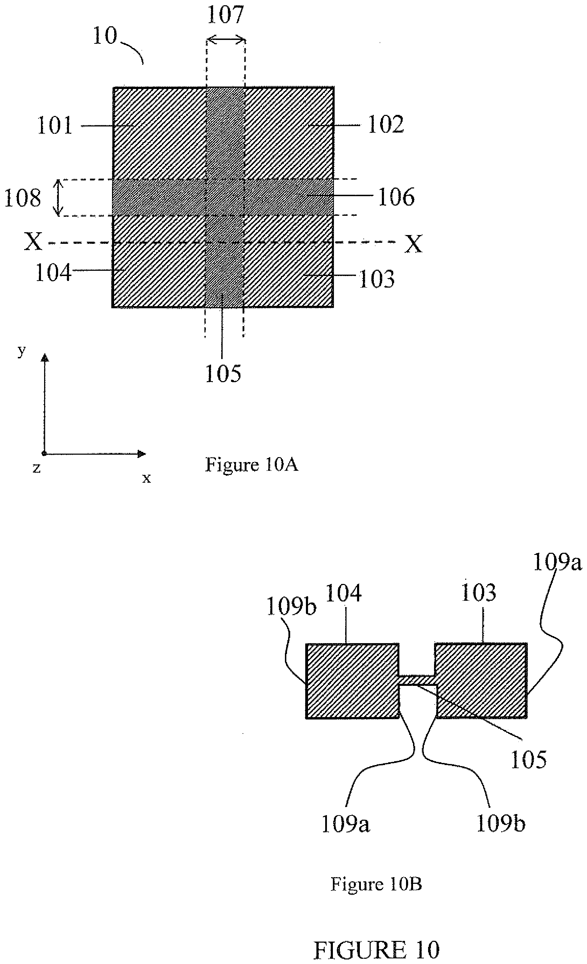

Il est maintenant fait référence aux figures 9 et 10 qui décrivent des modes de réalisation dans lesquels le boîtier d'encapsulation du dispositif selon l'invention contribue à la fonction de limitation de la flexion au sein du dispositif.

Il est tout d'abord fait référence aux figures 9A et 98 qui représentent, vu de dessus et vu en coupe selon la ligne AA, un autre mode de réalisation du dispositif selon l'invention.

Ainsi, le dispositif 9 est composé de quatre parties rigides 91, 92, 93 et 94 qui sont reliées entre elles par des parties flexibles 95, 96 et 97. Toutes ces parties sont à l'intérieur du boîtier d'encapsulation. Ces parties flexibles assurent la flexibilité du dispositif 9 par rapport à un axe quelconque parallèle à l'axe y, au niveau des zones 900, 901 et 902. Des pistes métalliques permettant de relier électriquement les quatre parties rigides sont présentes au niveau des zones flexibles et constituent des éléments sensibles à la flexion.

Dans l'exemple illustré à la figure 9, grâce à la forme du boîtier d'encapsulation, chacune des parties rigides 91 à 94 présente une section transversale (dans le plan xy) sensiblement carrée et chacune des parties flexibles 95 à 97 présente la forme d'une bande.

Ainsi, le boîtier d'encapsulation définit, au niveau de chaque partie rigide, une paroi 98 en regard d'une paroi 99, ces deux parois 98 et 99 étant sensiblement perpendiculaires à la partie flexible reliant deux parties rigides adjacentes.

Grâce à cette configuration, lorsqu'une flexion intervient par rapport à un axe parallèle à l'axe y au sein d'une zone 900, 901 ou 902, et que cette flexion a tendance à rapprocher l'une de l'autre les parois 98 et 99 (flexion vers le haut), ces dernières vont entrer en contact l'une avec l'autre dans la zone opposée à la partie flexible correspondante et, de ce fait, limiter la flexion du dispositif 9 par rapport à cet axe. Pour cela, il suffit que la hauteur H des parois 98 et 99 et l'espacement L entre les parois soient tels que L < πH et, de préférence, L < 2H.

On comprend que cette configuration présente l'avantage de ne pas nécessiter l'introduction d'éléments spécifiques au sein du dispositif pour limiter la flexion, cette fonction étant remplie par le boîtier d'encapsulation.

Il est maintenant fait référence à la figure 10 qui représente une variante de réalisation du dispositif illustré à la figure 9.

Dans ce mode de réalisation, le dispositif 10 est composé de quatre parties rigides 101, 102, 103 et 104 et de deux parties flexibles 105 et 106 qui relient deux à deux les parties rigides.

Dans l'exemple illustré à la figure 10, chacune des parties rigides 101 à 104 présente une section sensiblement carrée et chacune des parties flexibles 105, 106 présente la forme d'une bande, les deux parties flexibles formant une croix. Toutes ces parties du dispositif sont à l'intérieur d'un boîtier d'encapsulation.

Ainsi, chaque partie rigide comporte deux parois du boitier d'encapsulation 109a et 109b qui s'étendent sensiblement perpendiculairement aux parties flexibles.

Les parties flexibles 105 et 106 assurent la flexibilité du dispositif 10 par rapport à un axe quelconque parallèle à l'axe y dans la zone 107 et par rapport à un axe quelconque parallèle à l'axe x dans la zone 108.

La figure 10B qui est une vue en coupe selon l'axe X-X, permet de comprendre que les parois du boitier d'encapsulation 109a et 109b vont permettre de limiter la flexion du dispositif 10 à un rayon de courbure déterminé, lorsque ces deux parois vont entrer en contact du fait d'une flexion du dispositif 10 selon un axe parallèle à l'axe y au sein de la zone 107, que cette flexion se produise vers le haut ou vers le bas.

Les signes de référence insérés après les caractéristiques techniques figurant dans les revendications ont pour seul but de faciliter la compréhension de ces dernières et ne sauraient en limiter la portée.

## Revendications

1. Dispositif destiné à être fixé sur la peau d'un utilisateur, le dispositif étant sensiblement plan et **caractérisé en ce qu'**il comprend:
- au moins une zone flexible dans laquelle d'une part, le dispositif peut être fléchi par rapport à au moins deux axes situés dans le plan du dispositif, et dans laquelle d'autre part, sont situés des composants sensibles à la flexion,
- au moins deux moyens de limitation de la flexion (31, 32 ; 41,42 ; 51 à 54, 61 à 64 ; 71 à 76) à rayon de courbure déterminé, s'étendant dans des directions différentes, lesdits moyens de limitation de la flexion étant configurés pour limiter la flexion de ladite au moins une zone flexible du dispositif par rapport auxdits au moins deux axes situés dans le plan du dispositif.

2. Dispositif selon la revendication 1, dans lequel les moyens de limitation de la flexion (20, 30, 40, 50, 60, 70 ; 81, 82) comprennent une partie centrale (15, 25 ; 315, 325 ; 415, 425 ; 515, 525, 535, 545) allongée, de part et d'autre de laquelle s'étendent, dans le plan du dispositif, des parties en saillie (14, 24 ; 314, 324 ; 414, 424; 515, 524, 534, 544) définissant entre elles des renfoncements.

3. Dispositif selon la revendication 2, dans lequel la hauteur (H) des parties en saillie et la largeur (L) correspondant à l'espacement entre deux parties en saillie sont telles que L < πH et, de préférence, L < 2H.

4. Dispositif selon la revendication 2 ou 3, dans lequel les moyens de limitation de la flexion sont situés dans une partie sensiblement centrale du dispositif.

5. Dispositif selon l'une des revendications 2 à 4, dans lequel les moyens de limitation de la flexion (30, 40 ; 51 à 54 ; 60 ; 70) sont situés le long d'au moins une partie de la périphérie du dispositif.

6. Dispositif selon la revendication 5, dans lequel les moyens de limitation de la flexion (40) comportent une partie (41) située en périphérie du dispositif et une partie (42) s'étendant sensiblement au centre du dispositif.

7. Dispositif selon l'une des revendications 2 à 6, dans lequel la surface occupée par les moyens de limitation de la flexion est inférieure à 50% de la surface du dispositif et, de préférence, inférieure à 10% de cette surface.

8. Dispositif selon la revendication 1, dans lequel lesdits moyens de limitation de la flexion sont formés par un boîtier d'encapsulation du dispositif.

9. Dispositif selon la revendication 8, comprenant au moins deux parties rigides (91, 92, 93, 94 ; 101, 102, 103, 104) reliées par une zone flexible (95, 96, 97 ; 105, 106) dans laquelle sont situés les composants sensibles à la flexion, chacune desdites parties rigides comprenant au moins deux parois du boîtier d'encapsulation, les parois (98, 99 ; 109a, 109b) en regard de deux parties rigides adjacentes constituant lesdits moyens de limitation de la flexion par rapport à un axe s'étendant dans ladite zone flexible et parallèle auxdites parois.

10. Dispositif selon la revendication 9, comprenant une pluralité de parties rigides (91, 92, 93, 94) reliées entre elles deux à deux par une zone flexible (95, 96, 97) et s'étendant selon une direction déterminée.

11. Dispositif selon la revendication 9, comprenant au moins quatre zones rigides (101, 102, 103, 104) reliées entre elles par au moins deux zones flexibles (105, 106) s'étendant selon deux directions différentes.

12. Dispositif selon l'une des revendications 9 à 11, dans lequel la hauteur (H) desdites parois et la largeur (L) de l'espacement entre deux parois en regard sont telles que L < πH et , de préférence, L < 2H.

## Patentansprüche

1. Vorrichtung, die dazu bestimmt ist, an der Haut eines Benutzers befestigt zu werden, wobei die Vorrichtung im Wesentlichen eben und **dadurch gekennzeichnet ist, dass** sie umfasst:
- mindestens einen biegsamen Bereich, in dem einerseits die Vorrichtung in Bezug auf mindestens zwei in der Ebene der Vorrichtung befindliche Achsen gebogen werden kann, und in dem sich andererseits biegeempfindliche Komponenten befinden,
- mindestens zwei Mittel zum Begrenzen der Biegung (31, 32; 41, 42; 51 bis 54, 61 bis 64; 71 bis 76) mit bestimmtem Krümmungsradius, die sich in unterschiedlichen Richtungen erstrecken, wobei die Biegebegrenzungsmittel so konfiguriert sind, dass sie die Biegung des mindestens einen biegsamen Bereichs der Vorrichtung in Bezug auf die mindestens zwei in der Ebene der Vorrichtung befindlichen Achsen begrenzen.

2. Vorrichtung nach Anspruch 1, wobei die Biegebegrenzungsmittel (20, 30, 40, 50, 60, 70; 81, 82) einen länglichen Mittelteil (15, 25; 315, 325; 415, 425; 515, 525, 535, 545) umfassen, auf dessen zwei Seiten sich in der Ebene der Vorrichtung vorspringende Teile (14, 24; 314, 324; 414, 424; 515, 524, 534, 544) erstrecken, die zwischen sich Vertiefungen definieren.

3. Vorrichtung nach Anspruch 2, wobei die Höhe (H) der vorspringenden Teile und die Breite (L), die dem Abstand zwischen zwei vorspringenden Teilen entsprechen, so sind, dass L < πH und vorzugsweise L < 2H.

4. Vorrichtung nach Anspruch 2 oder 3, wobei sich die Biegebegrenzungsmittel in einem im Wesentlichen mittleren Teil der Vorrichtung befinden.

5. Vorrichtung nach einem der Ansprüche 2 bis 4, wobei sich die Biegebegrenzungsmittel (30, 40; 51 bis 54; 60; 70) entlang mindestens eines Teils des Umfangs der Vorrichtung befinden.

6. Vorrichtung nach Anspruch 5, wobei die Biegebegrenzungsmittel (40) einen Teil (41) umfassen, der sich am Umfang der Vorrichtung befindet, und einen Teil (42), der sich im Wesentlichen in der Mitte der Vorrichtung erstreckt.

7. Vorrichtung nach einem der Ansprüche 2 bis 6, wobei die von den Biegebegrenzungsmitteln eingenommene Fläche kleiner als 50 % der Fläche der Vorrichtung und vorzugsweise kleiner als 10 % dieser Fläche ist.

8. Vorrichtung nach Anspruch 1, wobei die Biegebegrenzungsmittel von einem Verkapselungsgehäuse der Vorrichtung gebildet werden.

9. Vorrichtung nach Anspruch 8, die mindestens zwei starre Teile (91, 92, 93, 94; 101, 102, 103, 104) umfasst, die durch einen biegsamen Bereich (95, 96, 97; 105, 106), in dem sich die biegeempfindlichen Komponenten befinden, miteinander verbunden sind, wobei jeder der starren Teile mindestens zwei Wände des Verkapselungsgehäuses umfasst, wobei die Wände (98, 99; 109a, 109b), die zwei angrenzenden starren Teilen zugewandt sind, die Mittel zur Begrenzung der Biegung in Bezug auf eine Achse darstellen, die sich in dem biegsamen Bereich und parallel zu den Wänden erstreckt.

10. Vorrichtung nach Anspruch 9, die eine Vielzahl von starren Teilen (91, 92, 93, 94) umfasst, die durch einen biegsamen Bereich (95, 96, 97) paarweise miteinander verbunden sind und sich in einer bestimmten Richtung erstrecken.

11. Vorrichtung nach Anspruch 9, die mindestens vier starre Bereiche (101, 102, 103, 104) umfasst, die durch mindestens zwei biegsame Bereiche (105, 106) miteinander verbunden sind, die sich in zwei unterschiedlichen Richtungen erstrecken.

12. Vorrichtung nach einem der Ansprüche 9 bis 11, wobei die Höhe (H) der Wände und die Breite (L) des Abstands zwischen zwei einander zugewandten Wänden so sind, dass L < πH und vorzugsweise L < 2H.

## Claims

1. A device intended to be fixed on the skin of a user, the device being substantially planar and **characterized in that** it comprise:
- at least one flexible zone in which the device can be bent relative to two axes situated in the plane of the device and in which flexure-sensitive components are situated,
- at least two flexure limiting means (31, 32; 41, 42; 51 to 54, 61 to 64; 71 to 76) to determined curve radius, extending along different directions, said flexure limiting means being configured to limit the flexure of said at least one flexible zone of the device relative to said at least two axes situated in the plane of the device.

2. The device according to claim 1, wherein the flexure limiting means (20, 30, 40, 50, 60, 70; 81, 82) comprise an elongated central part (15, 25; 315, 325; 415, 425; 515, 525, 535, 545), on either side of which protruding parts (14, 24; 314, 324; 414, 424; 515, 524, 534, 544) extend that define recesses between them.

3. The device according to claim 2, wherein the height (H) of the protruding parts and the width (L) corresponding to the space between the two protruding parts are such that L < πH, and preferably L < 2H.

4. The device according to claim 2 or 3, wherein the flexure limiting means are situated in a substantially central part of the device.

5. The device according to one of claims 2 to 4, wherein the flexure limiting means (30, 40; 51 to 54; 60; 70) are situated along at least part of the periphery of the device.

6. The device according to claim 5, wherein the flexure limiting means (40) comprise a part (41) situated at the periphery of the device and a part (42) extending substantially at the center of the device.

7. The device according to one of claims 2 to 6, wherein the surface occupied by said flexure limiting means is less than 50% of the surface of the device, and preferably less than 10% of the surface.

8. The device according to claim 1, wherein said flexure limiting means are formed by the encapsulating unit of the device.

9. The device according to claim 8, comprising at least two rigid parts (91, 92, 93, 94; 101, 102, 103, 104) connected by a flexible zone (95, 96, 97; 105, 106), in which flexure-sensitive components are situated, each of said rigid parts comprising at least two walls of the encapsulating unit, the walls (98, 99; 109a, 109b) opposite two adjacent rigid parts constituting means for limiting the flexure along an axis extending in said flexible zone and parallel to said walls.

10. The device according to claim 9, comprising a plurality of rigid parts (91, 92, 93, 94) connected to one another in pairs by a flexible zone (95, 96, 97) and extending along a determined direction.

11. The device according to claim 9, comprising at least four rigid zones (101, 102, 103, 104) connected to one another by at least two flexible zones (105, 106) extending in two different directions.

12. The device according to one of claims 9 to 11, wherein the height (H) of said walls and the width (L) of the spacing between two opposite walls are such that L < πH, and preferably L < 2H.
